# EUROPEAN PATENT APPLICATION

(11) **EP 2 431 002 A1**
(43) Date of publication of application: **21.03.2012**
(21) Application number: 11290415.6
(22) Date of filing: 15.09.2011
(51) Int. Cl.: A61B 18/20, A61B 18/00, A61B 18/22

(54) **Laser-based lipolysis**

(30) Priority: 16.09.2010 EP 10290495; 15.09.2010 US 383325 P; 14.09.2011 US 232130
(71) Applicant: Dornier MedTech Laser GmbH, 82234 Wessling (DE)
(72) Inventor: Rheinwald, Markus, 86916 Kaufering (DE); Milleret, René, 34000 Montpellier (FR)
(74) Representative: Grünecker, Kinkeldey, Stockmair & Schwanhäusser

(57) **Abstract**

The invention provides systems and methods for reducing adipose tissue in a homogenous manner by regulated delivery of energy to a treatment site, and more particularly, delivery of constant thermal energy which results in a reduction of adipose tissue with little or no damage to other adjacent tissue.

## Description

### CROSS-REFERENCE TO RELATED APPLICATIONS

This application claims priority to co-pending U.S. Provisional Patent Application No. 61/383,325 filed September 15, 2010 and European Patent Application No. 10290495 filed September 16, 2010. The contents of both priority applications are hereby incorporated in their entirety by reference.

### FIELD OF THE DISCLOSURE

The present invention is directed to systems and methods for reducing adipose tissue at a target site in a subject. The systems and methods of the present invention permit delivery of constant thermal energy to the treatment site, resulting in reduction of adipose tissue with improved homogeneity and little or no damage to adjacent tissue.

### BACKGROUND OF THE DISCLOSURE

The weight and contour of the human body is largely influenced by the amount and volume of adipose tissue, also referred to as body fat, in subcutaneous layers. For selected individuals, a higher volume of adipose tissue represents an unacceptable deviation from a desired body appearance.

Liposuction is a medical procedure for the removal of unwanted body fat. The procedure has been in relatively common use for more than thirty years. As originally adopted, liposuction involved the creation of small incisions in the skin, followed by the insertion of cannulas attached to a closed negative-pressure system allowing for aspiration of excess adipose tissue. The unwanted tissue was then collected in a receiving container and subsequently disposed of.

Although liposuction offered patients a new way of reducing unwanted body fat, the procedure as initially adopted was associated with considerable bleeding, pain and overall risk to the patient. In certain instances, the aesthetic result desired by the patient was not achieved, and in other instances, the patient's appearance was actually worsened.

Many of the problems associated with early liposuction resulted from the challenging structure of the target tissue. The layer of fat tissue underneath the skin is not homogeneous. Rather, collageneous septae connect the tissue layers on top and underneath the layer of fat tissue and, therefore, cross the layer of fat tissue. These septae cause increased resistance to the movement of the tube or needle used for suctioning the fat whenever the tip of the tube or needle is pushed against such a septum. In order to maintain a certain constancy in the speed of movement of the tube or needle, the operator was required to apply increased force to the movement, sufficient to overcome the increased resistance without significant change of the movement. The use of increased force created some degree of violence in the movements necessary for the completion of the procedure, which produced increased procedural trauma to the treatment site. This trauma was associated with considerable blood loss peri-operatively and pain post-operatively, prolonging recovery times. In addition, the skin covering the tissue removed remained stretched post-operatively, producing an unfavorable laxity in its appearance.

Liposuction has been improved considerably over the years, resulting in increased safety and effectiveness and diminishing side effects patients. It remains among the most common cosmetic surgical procedures.

One of the most important improvements was the use of local, as opposed, to general anesthesia. So-called tumescent anesthesia involves the application of local anesthesia, commonly in the form of large volumes of fluid containing a small amount of lidocaine and epinephrine. Tumescent anesthesia advantageously reduces topical pain sensation during the procedure, spatially buffers adjacent tissue structures from mechanical damage, and creates a medium for the sputtered debris of adipose cells and the contained fat to be suctioned out with underpressure through the needle or tube. The development of tumescent anesthesia also permitted the liposuction procedure to be performed on an outpatient basis.

Improvements have also been made to the devices used in the procedure. The cannulas originally used to remove the fat were large, i.e., with diameters in the range of 6 to 10 mm. Later, thinner (2mm and smaller) and longer needles or tubes were adopted, which reduced the invasiveness of the procedure by decreasing the size of the hole required to access the tissue layer of interest. Longer needles also permitted the operator to reach out to tissue regions far out in lateral distance from the entrance hole yet in the same target layer.

The longer needles or tubes also permitted the operator to scan over an area of interest within the target layer in the adipose tissue. The primary scan movement involves a forth-and-back movement of the needle or tube along its longitudinal extension to sequentially access a target amount of individual target volumes within the target tissue layer along the line of the longitudinal movement, the line having an extension from the minimal distance from the entry point of the needle or tube through the skin into the target layer to the maximum distance from this point. The minimal distance is determined by the minimal length of the portion of the tube or needle that is required to stay inside the target tissue layer such as not to inadvertently slip out of the entry point. The maximum distance is determined by the length of the tube or needle used and the extension of the target scan pattern in that direction with respect to the spatial arrangements of scan patterns in order to cover the target area to be treated to the degree that is maximally achievable. During that movement and with the underpressure applied to the tip of the tube or needle, the volume of fat in direct contact with the tip of the tube or needle at a given time will be suctioned into the tube or needle, hence to outside the body.

The secondary scan movement is to displace the line along which the tube is moved forth and back within the primary scan movement with an incremental azimuthal angle around the entry point within the target layer after one cycle of the primary scan movement (one movement forth and one movement back of the tip of the needle or tube). The degree of the incremental azimuthal angle applied in an individual patient is determined by the operator depending on the amount of fat to be removed, the rate at which the fat can be removed through the tube or needle, and the maximum distance of the line of the primary scan movement to the entry point that would then define a maximum radius. Complete coverage of the target area by the scan pattern is then achieved if the diameter of interaction of the tip of the tube or needle with the bodily fat equals the maximum radius multiplied with the incremental radian azimuthal angle of the secondary scan movement. A series of secondary scan movements would finally result in a fan-shape for the complete scan pattern. Overall azimuthal angles for the fan patterns are usually in the 30 to 60 degrees range.

Other improvements to the devices used in liposuction were focused on easing the burden on the operator. Liposuction can be a physically strenuous procedure for the operator. Vibrating or oscillating cannulas were developed to provide the movement typically provided manually by the operator to break down the membranes of adipose cells, thereby reducing operator fatigue and improving control over the procedure. Still other improvements to the procedure involved enhanced supportive processes. For example, methods of wrapping the treated body zone post-treatment were adopted.

A more fundamental improvement to the procedure involved the use of heat. Heat can be generated at the tip of the elongate applicator by applying different forms of energy, primarily electromagnetic radiation in the visible and infrared ranges of the wavelength spectrum. The energy delivered by the distal end of the elongate applicator is absorbed by the tissue volume adjacent to that distal end and then transformed into heat energy contained in that tissue volume. The amount of heat energy deposited results in an increase of the temperature of that tissue volume depending on the size of the volume and the specific heat capacity of the tissue in that volume.

The application of heat energy produces destruction of the membrane of the adipocytes, freeing fat from within the adipocytes to the intercellular space from which it can be evacuated more easily. The heat also liquefies or "melts" the fat, making it more viscous so as to ease the process of evacuation. The aqueous fluid of the tumescent anesthetic envelops the target layer of tissue treated and over a specific time acts as a heat sink to the local heating of the target tissue volume, which target volume cools down again by dissipation of the heat. However, once liquefied and at the same time surrounded with the aqueous tumescence fluid, the fat emulsifies with that liquid and remains easy to be evacuated upon cooling.

Using heat-assisted methods to liquefy the fat, it was possible to then leave the dissolved fat inside the body to permit bodily self-cleaning processes to act to remove it, optionally supported by various forms of mechanical manipulation of the area from outside (e.g., massage, lymphatic drainage). The natural process of fat removal leads to a more homogeneous appearance of the treated area, although it required additional procedures (e.g., the massage or lymphatic drainage) and additional time to achieve the final result.

In another variation of the method, heat energy has also been applied to the layer of skin above the target layer of fat tissue, producing a tightening of the skin stretched by the underlying volume of fatty tissue pre-operatively. This application of heat positively impacts skin laxity post-operatively.

With its highest density concentrated around the very tip of the tube or needle, the heat energy also helps the fiber tip to glide more smoothly around the septae between the tissue layers on top and underneath the target layer of fat tissue. This also reduces the resistance caused by these septae against the movement of the tip of that element. Consequently, the operator can reduce the force applied for the realization of the longitudinal movement, reducing the "operational violence" that contributes to the procedural trauma experienced by the patient. Furthermore, the heat applied to the septae leads to their slight contraction going in line with the reduction of height of the fat layer. The use of heat also produces a haemostatic effect, reducing the overall blood loss. The reduced procedural mechanical trauma and reduced bleeding together significantly improved the procedure and increased recovery time.

Yet, heat-assisted fat removal methods suffered certain challenges. The way the heat energy is commonly applied to the target area causes some inhomogeneities in the individual energies deposited to a specific volume of tissue, leading to inhomogeneous results, including both overtreatment or undertreatment. Overtreatment can result from applying too much energy, resulting in an increased rate of side-effects, some of them potentially irreversible, while undertreatment of specific volumes may result from the effort to avoid overtreatment of other volumes. These inhomogeneities can be intrinsic or extrinsic.

For example, as the secondary scanning movement is represented by an incremental angulation of the primary longitudinal movement of the tube or needle, more overlap of target tissue volumes will naturally occur for the volumes lying more proximal to the entry point of the tube or needle, producing an inhomogeneity. Another form of inhomogeneity results from the fact that a complete target zone on the body to be treated has in most cases a more complex form than just a fan. So, that complete target zone must be covered by laterally putting together multiple fan-shaped scan patterns to cover an area as similar in shape and extension as possible to it. However, a set of multiple fan-like patterns is rather prone to see overlaps as only two sides of the pattern are clearly defined by the first and the last line (primary scan movement) of that very pattern, producing another inhomogeneity.

As another example, inhomogeneities in the angular stepping (secondary scan movement) and inhomogeneous lengths between individual lines of forth-and-back movements (primary scan movement) may be produced. Inhomogeneities in the speed of the forth-and-back movement (primary scan movement) caused by either the doctor himself, as it is a non-standardized manual movement, or the natural acceleration and deceleration around the turning points and by the inhomogeneous resistance the tip of the needle or tube is experiencing due to the inhomogeneous nature of the tissue (adipose vs. collagenous; native vs. processed) may also result.

To overcome these inhomogeneities, various methods of treatment feedback have been introduced to indicate various parameters to the operator and/or the energy supplying device. For example, indication to the operator of the temperature of the tissue to be treated, which is either a natural tissue temperature, if not treated yet, or an elevated temperature, if pretreated within the same session, but the heat energy has not dissipated completely yet. This makes the respective tissue more sensitive to scan pattern overlaps resulting in overtreatment by overheating.

Another approach to the problem involves indication to the operator on whether the needle has covered a specific point within the layer to be treated such that the operator would spatially avoid overlapping. A still further approach has been to indicate to the laser device the temperature in the surrounding of the tip of the tube or the needle in order to adjust the power of the energy source so as to only supply the amount of power needed to reach a certain target temperature.

These methods address the inhomogeneities that can be produced using heat-assisted liposuction in a limited way only, however. There remains a need to provide systems and methods for the energy-assisted removal of body fat that offer improved efficacy and safety, and in particular, improved homogeneity in the energy delivered and the result achieved.

The description of exemplary embodiments of the invention will show in more detail what surprisingly different findings were made when applying an apparatus according to the exemplary embodiments and how this is an improvement over the traditional methods described above.

### SUMMARY OF THE INVENTION

The present invention is directed to systems and methods for the reduction or elimination of excess adipose tissue.

In one embodiment, a method of reducing adipose tissue from a target area is provided, the method comprising applying energy to the target area, rapidly detecting thermal feedback from the target area, and responding to the thermal feedback by regulating the energy delivered so as to maintain the temperature at the target area in a constant range. In some embodiments, the thermal feedback is radiation emitted by pyrolytic glowing. In some embodiments, the thermal feedback is detected within about 20 ms after the energy is applied. In some embodiments, the thermal feedback is detected within about 15 ms after the energy is applied. In some embodiments, the thermal feedback is detected within about 10 ms after the energy is applied. In some embodiments, the thermal feedback is detected by the device that delivers the energy to the target area. In some embodiments, the target area is a superficial or localized deposit of adipose tissue within the body of a subject.

In some embodiments, the method further comprises sensing the temperature at the surface of the body above the target area in order to generate a temperature profile. In some embodiments, the temperature profile is used to regulate the energy delivered. In some embodiments, the temperature is sensed by a temperature accessory on an operator's finger. In some embodiments, the temperature is sensed by a temperature indicator placed on the skin covering the target area.

### BRIEF DESCRIPTION OF THE DRAWINGS

Some embodiments of the disclosure may be understood by referring, in part, to the present disclosure and the accompanying drawings, wherein:
FIGURE 1 illustrates a view of a laser instrument for free-hand work according to a specific example embodiment of the disclosure;
FIGURE 2 illustrates a view of a laser instrument for endoscopic purposes according to a specific example embodiment of the disclosure;
FIGURE 3 illustrates a view of a radiation detector of the type indicated in FIGS. 1 and 2 according to a specific example embodiment of the disclosure; and
FIGURE 4 illustrates a flow diagram of a method according to a specific example embodiment of the disclosure.

### DETAILED DESCRIPTION

The present invention is directed to devices, systems and methods for reducing adipose tissue. The present invention permits delivery of constant thermal energy to the treatment target site, which improves the homogeneity of the result.

### I. Device

The present disclosure relates, in some embodiments, to a device for delivering energy to a target area in a regulated manner in order to reduce adipose tissue. The energy supplied by the device converts the adipose tissue to a form that can be more easily removed from the body, either by natural or assisted removal methods, or in some instances, both. An exemplary device that may be useful for this application is described in U.S. Patent No. 5,098,427 ("the '427 patent"), which is herein incorporated by reference in its entirety.

In one embodiment, the device delivers electromagnetic energy. In a particular embodiment, the device is a laser-emitting device. In a specific embodiment, the device is a laser-emitting device (e.g., a diode laser, a Nd:YAG laser, or any other useful laser known to one of skill in the art) having (i) a source of laser radiation; (ii) a guiding means (e.g., an optical light guide) configured to conduct radiation between the proximal end and distal end of the guiding means, the distal end being adapted to direct the laser radiation onto the target area (e.g., a biological tissue); (iii) a detector means for detecting radiation emitted from the target area (e.g. radiation emitted by pyrolytic glowing of the tissue), wherein the detector means generates an output signal in response thereto; and (iv) a regulator means, which responds to the output signal by either maintaining, increasing or decreasing the radiation emitted by the device such that the thermal energy emitted at the distal end is maintained at a constant temperature that is sufficiently high enough to convert the adipose tissue to a form that can be more readily removed from the body (e.g., an emulsified or liquefied form). The constant temperature is ideally high enough to emulsify or liquefy the adipose tissue and to deform the septae, yet low enough not to irreversibly damage or harm the adjacent tissue structures, including the septae.

The laser may operate/emit radiation at any useful wavelength known to one of skill in the art, including, but not limited to, wavelengths between about 0 to about 10⁶ nm, wavelengths between about 0 to about 10² nm, wavelengths between about 10² nm to about 10⁴ nm, wavelengths between about 10⁴ nm to about 10⁶ nm or wavelengths greater than about 10⁶ nm. The wavelengths may be about below 10¹ nm, about 10¹ nm, about 10² nm, about 10³ nm, about 10⁴ nm, about 10⁵ nm, about 10⁶ nm, any value in between these wavelengths, or greater than about 10⁶ nm. In one particular embodiment, the wavelength may be about 940 nm. In one particular embodiment, the wavelength may be about 10³ nm.

The detector means can be any suitable means to detect energy emitted by the target area. In one embodiment, the detector means has a photodiode (e.g., Siemens PBX 60 model or model BPW34 from Vishay Semiconductors) and a power-voltage converter (e.g., National Semiconductor mode LF356). Light impinging on the diode is converted to a voltage signal proportional to the light intensity with the amplification being adjusted by the resistor. In a particular embodiment, the radiation is detected in the spectral region of between about 0.3 and about 0.9 µm.

Similarly, the regulator means can be any suitable means to regulate the delivery of energy in response to the output signal. In one embodiment, the regulator means is an electronic control having a microcomputer assembly system (e.g., Siemens AMS-M6-A8 or model SAF-C167CR-LM from Infineon Technologies) which controls the laser by regulating its power in a conventional manner.

In one embodiment, the present invention is a system for delivering constant thermal energy to the target area in order to achieve the reduction in adipose tissue. The system includes both (i) the device described above to permit regulated delivery of thermal energy as well as (ii) a second temperature sensing device to permit the operator to detect the temperature of the body surface above the target tissue in an accurate and objective manner. The tissue thermal sensing device provides thermal feedback on the temperature of the external tissue volume being treated rather than on the guide or fiber tip.

In one embodiment, the temperature sensing device is an accessory provided on the operator's finger with a temperature indicator, such as a thermometer or a sheath located on the skin covering the treatment area with a visual indicator for the operator.

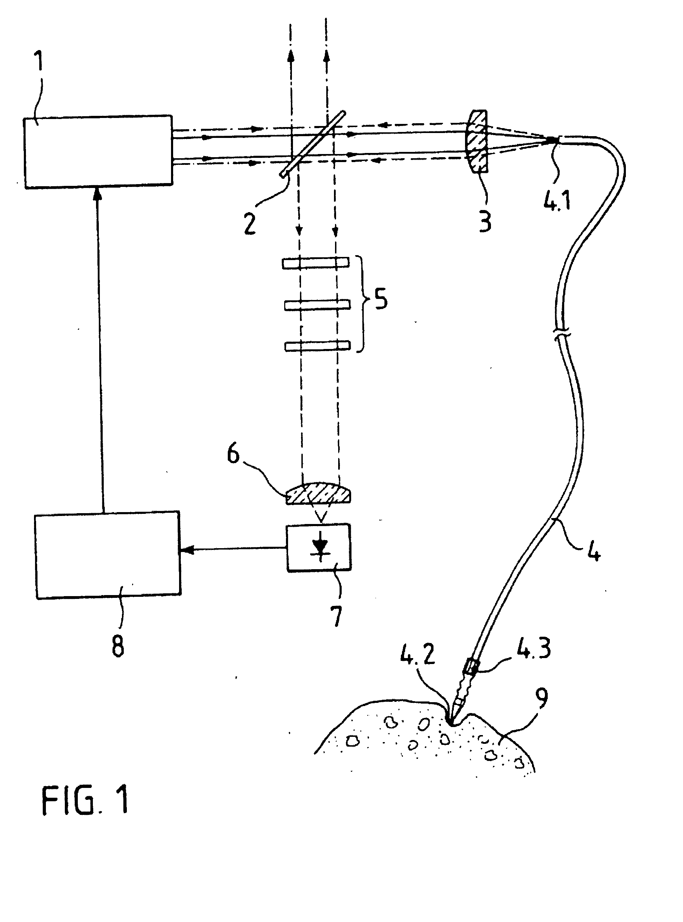

Referring now to the figures, in the embodiment illustrated in FIG. 1, the radiation of a medical laser (1) is coupled into the proximal end (4.1) of an optical fiber (4) by way of a wavelength-selective beam splitter (2) and a lens system (3). The laser instrument (1) is a Neodym YAG therapy laser with radiation that is at approximately 1 µm, that is, in the short infrared range. Beam splitter (2) is constructed as a wavelength-selective mirror, with the coatings selected such that the radiation of the Neodym YAG laser can pass through unimpaired, while the radiation emitted by the pump light sources on the laser side (20) (mainly in the visible range), as well as the radiation returned on the light guide side (22), are reflected in the spectral region between 0.3 and 0.9 µm. The radiation (21) reflected on the laser side (20) is diverted or absorbed in an unutilized manner, while the radiation (23) reflected on the light guide side, by way of a filter system (5) and another lens system (6), reaches a radiation detector (7), the output signal of which is analyzed in an electronic control circuit (8), and is used for controlling the laser (1). The latter may take place, for example, by regulating the power supply for the pump light source.

The therapy radiation coupled into the optical fiber (4) is aimed at the tissue (9) to be treated, for the purpose of which the distal end (4.2) of the light guide is surrounded by a handpiece (4.3). The distal end (4.2) of the optical fiber projects a few millimeters out of this handpiece (4.3) and is freed of its cladding (buffer) in the area of the most extreme tip.

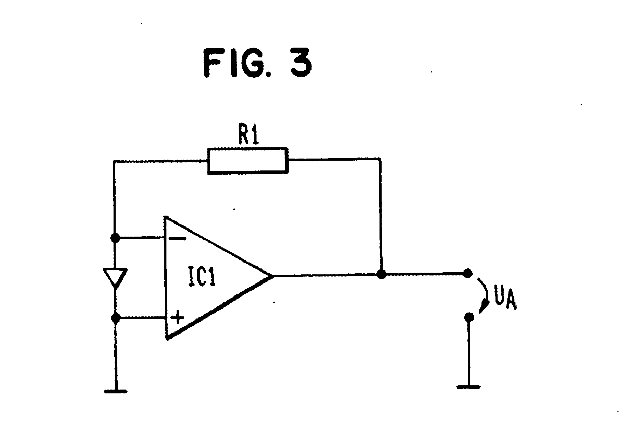

As illustrated in FIG. 3, radiation detector (3) is comprised of a photodiode D1 (such as for example, Siemens PBX 60 model) and a power voltage converter IC1 (such as National Semiconductor mode LF356). Light impinging on diode D1 is converted to a voltage signal U_{A} proportional to the light intensity, with the amplification being adjusted by the resistor R1. Electronic control unit (8), on the other hand, may consist of a microcomputer assembly system such as Siemens model AMS-M6-A8, which controls laser (1) by regulating its power supply in a conventional manner.

Referring again to FIG. 1, the radiation emitted during carbonization of the irradiated tissue, which includes the essential range of the visible spectrum, is received by the distal end (4.2) of the optical fiber, and is guided back by way of this optical fiber and impinges on the beam splitter (2). At the layer of the beam splitter (2) that has the wavelength-selective effect, the radiation (23) is deflected out of the beam path of the laser (1) in the direction of the radiation detector (7). In order to limit the received radiation to the so-called white-light region, the optical filter (5) is designed as a band-pass filter for the region between 0.3 and 0.9 µm, preferably between 0.4 and 0.8 . µm. For the spectral regions to be filtered out, the damping of the filter should be better than 10⁵.

In response to the signal received from radiation detector (7), electronic control unit (8) controls the output of laser (1) so as to prevent the received radiation output in the detector (7) from exceeding a predetermined value which corresponds to the destruction threshold of the light guide which is used.

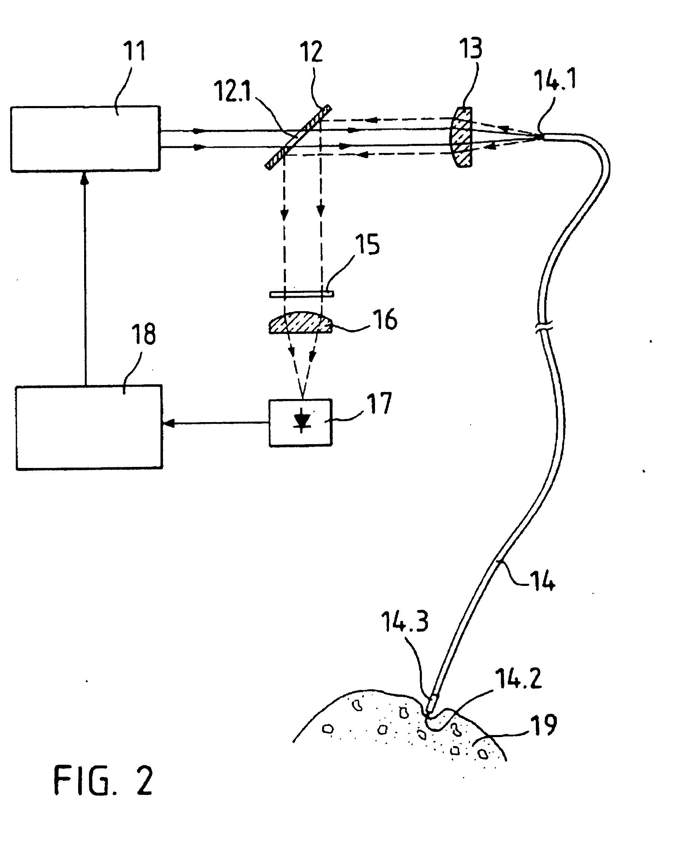

The embodiment according to FIG. 2 differs from the preceding embodiment mainly because of the fact that it is used for endoscopic purposes. For this purpose, the distal end of the optical fiber (14) has a cladding (14.3) that is visible in x-ray light. The beam splitter (12) between the laser (11) and the coupling-in lens system (13) in this case consists of a wide-band reflector, such as a metallic mirror, with an opening (12.1) in the center, through which the therapy radiation of the laser (11) can pass unimpaired. Thereafter, the radiation is coupled into the optical fiber (14) by way of the lens system (13). The light in the spectral region between 0.3 and 0.9 µm emitted by the pyrolytic glowing of at least partially carbonized biological tissue (19) is received in turn by the distal end (14.2) of the optical fiber (14), is guided back and, at the proximal end (14.1), is radiated with the full aperture angle of the fiber. The cross-section of the returned radiation beam (24) which is generated by the lens system (13) is larger than that of the laser radiation, and therefore impinges on the beam splitter (12) outside the opening (12.1). The guided-back radiation is thus reflected at the beam splitter (12) and, by way of a filter (15), which suppresses the spectral region of the laser radiation and by way of a lens system (16), reaches radiation detector (17) which, analogously to the preceding embodiment, emits a signal to a control device (18) which, in turn, controls the laser (11) with respect to its radiation output.

A very hot temperature-controlled so-called "hot tip" may be produced by the fact that a material, such as carbon, which absorbs laser light is embedded into the core layer of the optical fiber (14) at its distal end (14.2). In the absence of air, it only glows up, and its irradiation is maintained at a constant value by means of the control loop.

### II. Methods of Use

The present invention also includes a method for reducing adipose tissue in a subject. The method involves the delivery of thermal energy in a regulated manner to a target area in a patient and more specifically, the delivery of constant thermal energy to the target area. The energy delivered converts the adipose tissue present in the target area into a form more readily removable from the body, which removal can occur by active (e.g., suction) or passive means (e.g., biological processes), or a combination of both. The delivery of constant thermal energy according to the invention provides an advantageous homogeneity to energy deposited and the result achieved by the fat removal procedure.

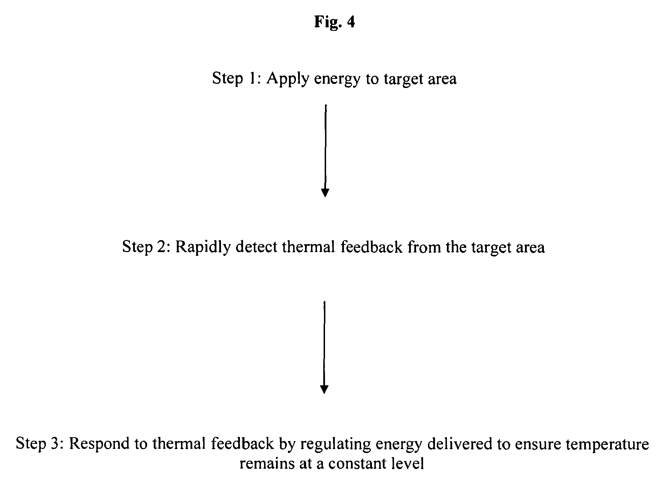

In one embodiment and as illustrated in Figure 4, the present invention is a method of reducing adipose tissue from a target area, comprising (i) applying energy to the target area; (ii) rapidly detecting thermal feedback from the target area; (iii) responding to the thermal feedback by regulating the energy delivered so as to maintain the temperature at the target area at a constant level (e.g. in a constant range or at a constant value).

### A. Target Area

The method of the present invention can be used to reduce excess or unwanted adipose tissue in a subject and more particularly, to reduce adipose tissue in one or more target areas within the subject. The subject may be any human or non-human animal. The target area may be any area of the subject's body understood by one of skill in the art to be appropriate for treatment with a fat removal procedure. For example, the method can be used to reduce or eliminate superficial or localized fat deposits in the thighs (including inner and outer thighs), the abdomen (including upper and lower abdomen), the lower back, the hips, the flank, the face, neck, upper arms, the sides of the knees, and the lower legs.

When sufficient energy is applied, the fat deposit previously present within the target area is converted into a form more readily removable from the body. The fat deposit or adipose tissue may be, for example, emulsified or liquefied. The amount of adipose tissue to be removed from the site is determined according to best practices know to those of skill in the art.

### B. Delivery of and Response to the Application of Energy to the Target Area

The method of the present invention involves regulation of energy delivered to the target site in order to reduce adipose tissue. Regulation of the energy delivered permits the thermal energy to be deposited in a more homogenous way, improving the homogeneity of the result, i.e., the reduction of unwanted adipose tissue and the physical appearance of the area of the body where adipose tissue has been removed.

The delivery of thermal energy is regulated according to the present invention by a feedback mechanism that (i) detects thermal energy emitted by the target site ("thermal feedback") and (ii) responds to that feedback by maintaining or adjusting (either up or down) the amount of energy delivered to the target site. For example, if the thermal energy emitted by the target site is too great relative to a pre-established standard, the amount of energy delivered to the target sight will be reduced. Correspondingly, if the thermal energy emitted by the target site is low relative to a pre-established standard, the amount of energy delivered to that site will be increased. In one embodiment, the method of the present invention permits delivery of constant thermal energy to the target area (e.g., a biological tissue).

In one embodiment, the thermal energy or temperature is sufficiently high to convert the adipose tissue into a form more readily removed from the body (e.g., about 45 to about 55 degrees centigrade), but not so high as to irreversibly damage or harm the septae or adjacent tissue structures such as the dermis or epidermis (e.g., greater than about 70 degrees centigrade). The delivery of constant thermal energy also allows the epidermis or the skin at the treatment area to properly contract after the procedure without any side effects, such as negative effects on pigmentation.

In certain exemplary embodiments, the device is designed to allow different constant temperatures for different applications.

In one embodiment, the energy delivered is electromagnetic energy (e.g., light) at a certain power. The electromagnetic energy can be delivered by an appropriate console, such as a laser. The electromagnetic energy is guided from the console to the target tissue via an appropriate guiding means, such as an optical light guide. The electromagnetic energy is then emitted from the distal end of the guiding means and targeted to the tissue volume to be treated. In the case of laser liposuction or laser lipolysis, the distal end of the optical light guide generally will be in contact with the target tissue volume inside the body.

The energy delivered to the target area or tissue is then absorbed by specific tissue components (such as water, hemoglobin, collagen, fat, etc.) within the volume irradiated. The amount of energy delivered may be any useful amount known to one of skill in the art including, but not limited to, between about 0 to about 12,000 J, between about 0 to about 3,000 J, between about 3,000 J to about 6,000 J, between about 6,000 J to about 9,000 J, between about 9,000 J to about 12,000 J, or greater than 12,000 J. The amount of energy delivered may be about below 1,000 J, about 1,000 J, about 2,000 J, about 3,000 J, about 4,000 J, about 5,000 J, about 6,000 J, about 7,000 J, about 8,000 J, about 9,000 J, about 10,000 J, about 11,000 J, about 12,000 J, any value in between these values, or greater than about 12,000 J. The size of the volume within which the electromagnetic energy will be absorbed depends on the absorption properties of that volume (e.g., character, properties, and concentration of absorbants contained in the volume). The electromagnetic energy is transformed into heat energy carried by the bodily absorbants. The heat energy generated out of the transformation from the electromagnetic energy is transported to volumes adjacent to the absorbants; the process being ruled by various heat energy transport mechanisms; the volumes including tissue in most cases, but also e.g., the tumescent medium. The application of energy and the transformation of that energy into heat energy produces changes to the biological tissue on both microscopic and macroscopic levels due to the heat energy applied there, the amount, speed and character of change being ruled by a function of the amount of heat energy the tissue is exposed to and the time it is exposed to the heat. The transportation of heat energy can occur in parallel with these changes.

Due to the high density of heat energy generated around the distal tip of the laser fiber, the tissue surrounding it may achieve such high temperatures that it will carbonize and stick to the fiber tip. After its generation, this charred tip will absorb almost all the laser light energy coming to it from the laser side, and the light energy will be transformed into heat energy within that tip. In certain exemplary embodiments, the tip has a small volume of about 1 mm³ or less and will therefore achieve very high temperatures able to emit light with a continuous spectrum in the visible light spectrum. With the fiber tip moved along longitudinal lines, the heat energy generated in the tip will be transported into the volumes surrounding this line, continuously taking up the heat energy which is generated by irradiating the charred fiber tip from the laser side. Then, this heat energy arriving at those volumes will cause the change of the tissue characteristics. Specifically, irradiation of the charred tissue stuck to the distal tip of the fiber with the light energy coming out of the laser will be followed by absorption of the light energy within that volume of charred tissue sticking to the distal tip. The light energy is then transformed into heat energy, which is then transported to volumes adjacent to the volume of charred tissue at that given time (with simultaneous longitudinal movement of that volume of charred tissue). Changes will occur in the biological tissue on both microscopic and macroscopic levels due to the heat energy applied there, the amount, speed and character of change being ruled by a function of the amount of heat energy the tissue is exposed to and the time it is exposed to the heat.

In one embodiment, the method of the present invention gathers thermal feedback before the impact of the heat energy on the target area becomes significantly advanced. In a particular embodiment, thermal feedback is sensed before heat energy is transported to volumes adjacent to the absorbents or before changes occur to the biological tissues. The method of the present invention is advantageous over feedback mechanisms that detect and response to thermal feedback only after heat energy transport or after biological changes have occurred, which is longer than this time, and potentially too long to have a beneficial effect for the immediate incremental power needed to be supplied to achieve a constant temperature at the fiber tip. Fast or rapid thermal feedback beneficially avoids excessive temperatures at the fiber tip which can be harmful to tissue volumes including fat, skin, and septae, potentially resulting in irreversible damage.

In one embodiment, the thermal feedback is gathered within about 40 ms after the energy is applied initially. In another embodiment, the thermal feedback is gathered within about 35 ms, about 30 ms, about 25 ms, about 20 ms, about 15 ms, about 10 ms, or about 5 ms after the energy is initially applied. In a particular embodiment, the time span between the initial application of energy and the detection or gathering of thermal feedback is about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 ms.

The radiation may be detected in any spectral region known to be useful to one of skill in the art. In general, the spectral region is between about 300 nm to about 11,000 nm. In certain embodiments, the spectral region is between about 300 nm to about 1,100 nm, between about 1,100 nm to about 2,100 nm, and/or between about 2,100 nm to about 11,000 nm. In the sub-range of between about 300 nm to about 1,100 nm, more specific ranges can be between about 300 nm to about 800 nm, between about 800 nm to about 1,000 nm, and/or between about 800 nm to about 1,100 nm, and in particular, the wavelengths of about 810 nm, about 940 nm, about 980 nm, and/or about 1,064 nm can be used. In the sub-range of between about 1,100 nm to about 2,100 nm, more specific ranges can be between about 1,100 nm to about 1,500 nm, between about 1,300 nm to about 1,400 nm, between about 1,400 nm to about 1,500 nm, and/or between about 1,900 nm to about 2,100 nm.

Certain measures of success of the method would largely depend on the time cycle needed for the tissue to reach the end temperature responsible for the final effect plus the reaction time for the thermal sensor plus the reaction time for the energy source to adjust the power to the appropriate value. Methods of the present invention are advantageous over feedback mechanisms wherein temperature sensors on the tip of the needle or the tube have a longer reaction time, such that inhomogeneities described previously are not flattened fast enough due to the speed of the movement required to timely complete the procedure. In addition, feedback mechanisms prior to the present invention cannot address inhomogeneities that require adapting the power almost instantaneously in order to resect collagenous structures different from the adipose tissue within the target layer.

The methods of the present invention address the more static inhomogeneities discussed above. Specifically, the method of the present invention addresses inhomogeneities that result from the fact that a complete target zone on the body to be treated has in most cases a more complex form than just a fan as well as inhomogeneous lengths between individual lines of forth-and-back movements (primary scan movement).

### C. Removal of Treated Adipose Tissue

As a result of the application of energy according to the methods disclosed herein, excess or unwanted adipose tissue present in the target area is converted to a removable form of fat. In one embodiment, the adipose tissue is liquefied or emulsified. It may then be removed from the body by a natural process, an assisted process or a combination of both. In one embodiment, the removable fat is removed by natural processes, i.e., the fat is carried away by the lymphatic system. In another embodiment, the fat is removed from the body by an assisted process. Assisted process may include, without limitation, suction or the application of mechanical manipulation of the body zone from outside (e.g., massage, lymphatic drainage).

### D. Additional Methods

The method of the present invention involves reducing adipose tissue at a target site by the application of regulated or constant thermal energy. It can be combined with other methods, including methods used to prepare the patient for the procedure or to support recovery from the procedure, which methods would be understood to one of skill in the art. It can also be combined with methods to enhance the feedback to the operator to ensure that the energy is provided in an optimized manner.

In one embodiment, the method of the present invention is used in combination with any appropriate form of anesthesia, including tumescent anesthesia or general anesthesia. Methods of anesthesia are familiar to those of skill in the art.

As another example, supportive therapies are used subsequent to the application of energy. For example, in one embodiment, the treatment site is wrapped to promote healing. Any method and/or material known to one of skill in the art may be used for this purpose, including, but not limited to bandages and compression garments.

In another embodiment, heat energy is also applied to the layer of skin situated on top of the target layer of fat tissue to improve the appearance of the skin once the fat is removed.

In a further embodiment, the method of the present invention is combined with one or more additional methods to monitor the delivery of energy to the target area. In one embodiment, the method is combined with a method of monitoring the temperature of the target area. Specifically, this additional method involves sensing the temperature of treated tissue on the outside of a patient's body. This temperature information can be used as an additional source of data to regulate the emitted laser energy according to the basic method.

As will be understood by those skilled in the art who have the benefit of the instant disclosure, other equivalent or alternative compositions, devices, methods, and systems for reducing adipose tissue can be envisioned without departing from the description contained herein. Accordingly, the manner of carrying out the disclosure as shown and described is to be construed as illustrative only.

### EXAMPLES

Some specific example embodiments of the disclosure may be illustrated by one or more of the examples provided herein.

### EXAMPLE 1:

A patient is treated to reduce or eliminate excess or unwanted adipose tissue. Tumescent anesthesia is administered to the treatment area prior to the procedure and the device is then used to apply energy to a target region within the body. The device targets electromagnetic energy to the target region which is then converted to thermal energy in the target area. Thermal feedback is generated in response to the application of the laser energy in the form of pyrolytic glowing of biological tissue within the target site, which glowing is then detected by the working end of the optical light guide. As the thermal feedback exceeds or falls below an established target temperature, the delivery of energy to the target site is regulated either up or down to maintain the delivery of thermal energy at a constant level. The continued delivery of thermal energy liquefies the adipose cells and tissue, which liquid fat is then removed by permitting the body's own natural processes. Upon completion of treatment with the device, the treatment area is then wrapped using bandages or compression garments to promote healing.

The method of the present invention can be further realized with the following embodiments:
1. A method of reducing adipose tissue from a target area, comprising (i) applying energy to the target area; (ii) rapidly detecting thermal feedback from the target area; (iii) responding to the thermal feedback by regulating the energy delivered so as to maintain the temperature at the target area in a constant range.
2. The method of embodiment 1, wherein thermal feedback is radiation emitted by pyrolytic glowing.
3. The method of embodiment 1 or 2, wherein the thermal feedback is detected within about 20 ms after the energy is applied.
4. The method of one of embodiments 1 to 3, wherein the thermal feedback is detected within about 15 ms after the energy is applied.
5. The method of one of embodiments 1 to 4, wherein the thermal feedback is detected within about 10 ms after the energy is applied.
6. The method of one of embodiments 1 to 5, wherein the thermal feedback is detected by the device that delivers the energy to the target area.
7. The method of one of embodiments 1 to 6, wherein the target area is a superficial or localized deposit of adipose tissue within the body of a subject.
8. The method of one of embodiments 1 to 7, further comprising sensing the temperature at the surface of the body above the target area in order to generate a temperature profile.
9. The method of embodiment 8, wherein the temperature profile is used to regulate the energy delivered.
10. The method of one of embodiments 8 or 9, wherein the temperature is sensed by a temperature accessory on an operator's finger.
11. The method of one of embodiments 8 to 10, wherein the temperature is sensed by a temperature indicator placed on the skin covering the target area.

The device of the present invention can be further realized with the following embodiments:
12. A laser-based lipolysis system for reducing adipose tissue of a patient while not or only minimally harming adjacent tissue structure, in particular septae, the system comprising:
   a laser light emitting console,
   an optical light guide coupled to said console and having a working end, and
   a regulation means for regulating the emitted laser energy to be high enough for reducing the adipose tissue, and to be low enough for not or only minimally harming adjacent tissue structures,
   wherein the regulation means is situated in the laser light emitting console.
13. A laser-based lipolysis system comprising:
   a laser light emitting console,
   an optical light guide coupled to said console and having a working end, and
   a regulation means for regulating the emitted laser energy to be high enough for reducing the adipose tissue, and to be low enough for not or only minimally harming adjacent tissue structures,
   wherein the regulation means is situated in the laser light emitting console, and wherein the system is used for reducing adipose tissue of a patient while not or only minimally harming adjacent tissue structure, in particular septae.
14. The laser-based lipolysis system of embodiments 12 or 13, wherein the regulation means comprises a radiation detector for detecting back guided radiation in a spectral region of between 0.3 and 0.9 µm.
15. The laser-based lipolysis system of one of the preceding embodiments, wherein the regulation means comprises a radiation detector for detecting radiation of a pyrolytic glowing of biological tissue on the working end of the optical light guide.
16. The laser-based lipolysis system of one of the preceding embodiments, wherein the regulation means is a regulation means for keeping a temperature of thermal energy emitted at the working end of the optical light guide constant.
17. The laser-based lipolysis system of one of the preceding embodiments, wherein the regulation means has a short time frame feedback mechanism, preferably for short time frames of up to 20 ms, more preferably for short time frames of up to 10 ms.
18. The laser-based lipolysis system of one of the preceding embodiments, for deforming the septae.
19. The laser-based lipolysis system of one of the preceding embodiments, for reducing adipose tissue while not or only minimally harming adjacent tissue structures of dermis or epidermis.
20. The laser-based lipolysis system of one of the preceding embodiments, for denaturing or emulsifying adipose tissue by laser light.
21. The laser-based lipolysis system according to one of the preceding embodiments comprising a tissue temperature sensing device for sensing a temperature of treated tissue on the outside of a patient's body.
22. The laser-based lipolysis system of embodiment 21, wherein the temperature sensing device complements the regulation means.
23. The laser-based lipolysis system of embodiments 21 or 22, wherein the temperature sensing device takes the form of an accessory that can be provided on an operator's finger.
24. The laser-based lipolysis system of embodiments 21 to 23, wherein the temperature sensing device comprises a temperature indicator, preferably a thermometer or a sheath that can be located on skin covering the treatment area.

## Claims

1. A laser-based lipolysis system comprising:
a laser light emitting console (1, 11),
an optical light guide (4, 14) coupled to said console and having a working end (4.2, 14.2), and
a regulation means (8, 18) for regulating the emitted laser energy,
wherein the regulation means (8, 18) comprises a radiation detector (7, 17) detecting radiation of a pyrolytic glowing of biological tissue on the working end of the optical light guide, and receiving said radiation conducted from said working end (4.2, 14.2) through said optical light guide (4, 14),
wherein the system is for reducing adipose tissue of a patient.

2. The laser-based lipolysis system of claim 1, **characterized in that** the radiation detector is capable of detecting back guided radiation in a spectral region of between 0.3 µm and 0.9 µm.

3. The laser-based lipolysis system of claims 1 or 2, **characterized in that** the radiation detector is capable of detecting radiation of pyrolytic glowing of biological tissue on the working end (4.2, 14.2) of the optical light guide (4, 14).

4. The laser-based lipolysis system of one of the preceding claims, **characterized in that** the regulation means (8, 18) is for keeping a temperature of thermal energy emitted at the working end (4.2, 14.2) of the optical light guide (4, 14) constant, preferably not greater than about 70° C, and in particular in the range of about 45° C to about 55° C.

5. The laser-based lipolysis system of one of the preceding claims, **characterized in that** the regulation means (8, 18) has a short time frame feedback mechanism of up to 40 ms, preferably for time frames up to 20 ms, more preferably for short time frames up to 10 ms, and more preferably for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 ms.

6. The laser-based lipolysis system of one of the preceding claims, **characterized in that** the system is for reducing adipose tissue while not or only minimally harming adjacent tissue structure, in particular of dermis or epidermis or septae.

7. The laser-based lipolysis system of one of the preceding claims, **characterized in that** a beam divider (2, 12) is provided between said optical light guide (4, 14) and said laser light emitting console (1, 11), said beam divider leading emitted radiation from said optical light guide to impinge on said radiation detector means (7, 17).

8. The laser-based lipolysis system according to claim 7, **characterized in that** said beam divider (2, 12) has the property that it transmits laser radiation from said laser light emitting console and reflects laser radiation from said laser light emitting console, and is so oriented that reflected radiation from said optical light guide (4, 14) impinges on said radiation detector means (7, 17).

9. The laser-based lipolysis system of one of the preceding claims, **characterized in that** the system is adapted to deliver energy to the target area of a tissue at an amount of about below 1000 J, 2000 J, 3000 J, 4000 J, 5000 J, 6000 J, 7000 J, 8000 J, 9000 J, 10000 J, 11000 J, or 12000 J, or greater than about 12000 J.

10. The laser-based lipolysis system of one of the preceding claims, **characterized in that** the spectral region of the laser light emitted by said console is between about 300 nm to about 11000 nm, between about 1100 nm to about 2100 nm, between about 2100 nm to about 11000 nm, between about 300 nm to about 800 nm, between about 800 nm to about 1000 nm, between about 800 nm to about 1100 nm, between about 1100 nm to about 1500 nm, between about 1300 nm to about 1400 nm, between about 1400 nm to about 1500 nm, or between about 1900 nm to about 2100 nm, and in particular the wavelength is of about 810 nm, about 940 nm, about 980 nm, and/or about 1064 nm.

11. The laser-based lipolysis system of one of the preceding claims, further comprising a tissue temperature sensing device for sensing a temperature of the target area on the outside of a patient's body, in particular for regulating the emitted laser energy.

12. The laser-based lipolysis system according to claim 11, **characterized in that** the temperature sensing device comprises a temperature indicator, preferably a thermometer or sheath that can be located on skin covering the target area.

13. The laser-based lipolysis system of one of claims 11 or 12, **characterized in that** the temperature sensing device takes the form of an accessory that can be provided on an operator's finger.

14. Use of the laser-based lipolysis system of one of the preceding claims for manufacturing a system for use in a method of reducing adipose tissue from a target area.
